Europäisches Patentamt

European Patent Office

Office européen des brevets

(11) Publication number: **0 117 694**
**A1**

(12) **EUROPEAN PATENT APPLICATION**

(21) Application number: **84301049.7**

(22) Date of filing: **17.02.84**

(51) Int. Cl.³: **C 12 N 15/00, C 12 R 1/19**

(30) Priority: **25.02.83 US 470015**
**29.07.83 US 518602**

(43) Date of publication of application: **05.09.84**
**Bulletin 84/36**

(84) Designated Contracting States: **BE CH DE FR GB IT LI NL SE**

(71) Applicant: **THE UPJOHN COMPANY, 301 Henrietta Street, Kalamazoo, Michigan 49001 (US)**

(72) Inventor: **Manis, Jack Jay The Upjohn Company, 301 Henrietta Street, Kalamazoo Michigan 49001 (US)**
Inventor: **Olsen, Mary Kathryn The Upjohn Company, 301 Henrietta Street, Kalamazoo Michigan 49001 (US)**

(74) Representative: **Perry, Robert Edward et al, GILL JENNINGS & EVERY 53-64 Chancery Lane, London WC2A 1HN (GB)**

(54) **Plasmids and plasmid vectors.**

(57) Novel plasmid vectors have been prepared and transformed into suitable hosts from which they can be retrieved and then used as cloning vehicles. The plasmids are named pUC1072, pUC1075, pUC1078, pUC1079 and pUC1080. Advantageously, these plasmids are shuttle vectors which can be used to clone genes in E. coli and Streptomyces.

EP 0 117 694 A1

# PLASMIDS AND PLASMID VECTORS

The development of plasmid vectors useful for recombinant DNA genetics among microorganisms is well known.  This technology is now being applied to industrially important microorganisms of the genus Streptomyces.   Pertinent references to the Streptomyces work are as follows:

(1)  Bibb, M. J., Ward, J. M., and Hopwood, D. A.: Transformation of plasmid DNA into Streptomyces at high frequency.   Nature 274 (1978) 398-400.

(2)  Bibb, M.J., Schottel, J.L. and Cohen, S.N.:  A DNA cloning system for interspecies gene transfer in antibiotic-producing Streptomyces.   Nature 284 (1980) 526-531.

(3)  Bibb, M.J. Ward, J.M., Kieser, T., Cohen, S.N., and Hopwood, D.A.:  Excision of chromosomal DNA sequences from Streptomyces coelicolor forms a novel family of plasmids detectable in Streptomyces lividans.  Mol. Gen. Genet. 184 (1981) 230-240.

(4)  Bibb, M.J. and Cohen, S.N.: Gene expression in Streptomyces: construction and application of promoter-probe plasmid vectors in Streptomyces lividans.  Mol. Gen. Genet. 187 (1982) 265-277.

(5)  Chater, K.F., Bruton, C.J., Springer, W. and Suarez, J.E.: Dispensible sequences and packaging constraints of DNA from Streptomyces temperate phage C31.  Gene 15 (1981) 249-256.

(6)  Chater, K.F., Bruton, C.J., King, A.A. and Suarez, J.E.:  The expression of Streptomyces and Escherichia coli drug-resistance determinants cloned into the Streptomyces phage C31.  Gene 19 (1982) 21-32.

(7)  Chater, K.F., Hopwood, D.A., Kieser, T., and Thompson, C.J.: Gene cloning in Streptomyces.  In Current Topics in Microbiology and Immunology, Vol. 96, (Hofschneider, P.H. and Goebel, W. eds), pg 69-96, Springer-Verlag, Berlin (1982).

(8)  Kieser, T., Hopwood, D.A., Wright, H.M., and Thompson, C.J.: pIJ101, a multi-copy broad host-range Streptomyces plasmid : functional analysis and development of DNA cloning vectors. Mol.

Gen. Genet. <u>185</u> (1982) 223-238.    <span>- 2 -</span>    4062.1

<span>0117694</span>

(9)  Richardson, M.A., Mabe, J.A., Beerman, N.E., Nakatsukasa, W.M. and Fayerman, J.T.:  Development of cloning vehicles from the <u>Streptomyces</u> plasmid pFJ 103. Gene, <u>20</u> (1982) 451-457.

(10) Schottel, J.L., Bibb, M.J., and Cohen, S.W.: Cloning and expression in <u>Streptomyces lividans</u> of antibiotic resistance genes derived from <u>Escherichia coli</u>.  J. Bacteriol. <u>146</u> (1981) 360-368.

(11) Suarez, J.E. and Chater, K.F.:  DNA cloning in <u>Streptomyces</u>: a bifunctional replicon comprising pBR322 inserted into a <u>Streptomyces</u> phage.  Nature <u>286</u> (1980) 527-529.

(12) Thompson, C. J., Ward, J. M., and Hopwood, D. A.:  "DNA cloning in <u>Streptomyces</u>:  resistance genes from antibiotic-producing species.  Nature <u>286</u> (1980) 525-527.

(13) Thompson, C.J., Kieser, T., Ward, J.M., and Hopwood, D.A.: Physical analysis of antibiotic-resistance genes from <u>Streptomyces</u> and their use in vector construction.  Gene <u>20</u> (1982) 51-62.

(14) Thompson, C.J., Skinner, R.H., Thompson, J., Ward, J.M., Hopwood, D.A. and Cundliffe, E.:  Biochemical characterization of resistance determinants cloned from antibiotic-producing streptomycetes.  J. Bacteriol. <u>151</u> (1982) 678-685.

(15) Thompson, C.J., Ward, J.M., and Hopwood, D.A.:  Cloning of antibiotic resistance and nutritional genes in streptomycetes. J. Bacteriol. <u>151</u> (1982) 668-677.

Plasmid pUC6 was isolated from <u>Streptomyces espinosus</u> biotype 23724a, NRRL 11439.  Its characteristics have been described in U.S. Patent 4,273,875.

Plasmids pBR322 and pBR325 are well known plasmids which can be obtained from <u>E. coli</u> RR1, NRRL B-12014 and NRRL B-15295, respectively.  The restriction endonuclease maps for plasmids pBR322 and pBR325 are published [Sutcliff, J. G. "pBR322 restriction map derived from the DNA sequence:  accurate DNA size markers up to 4361 nucleotide pairs long."  Nucleic Acids Research <u>5</u>, 2721-2728, 1978] and [Bolivar, F.:  Construction and characterization of new cloning vehicles, III.  Derivatives of plasmid pBR322 carrying unique <u>EcoRI</u> sites for selection of <u>EcoRI</u> generated recombinant molecules.  Gene <u>4</u> (1978) 121-136.], respectively.  These maps are incorporated herein by reference to the above publications.

## BRIEF SUMMARY OF THE INVENTION

Plasmid pUC1072 is an ampicillin resistance ($Ap^R$) plasmid that is obtained by the in vitro covalent linkage of the E. coli plasmid pBR322 to the S. espinosus plasmid pUC6. Plasmid pUC1078 is a derivative of pUC1072 having a TaqI fragment carrying the ribosome binding side and structural gene for E. coli chloramphenicol acetyl-transferase (cat) in the orientation that allows expression chloramphenicol resistance ($Cm^R$) from the anti-tet promoter of pBR322 [Covarrubias, L. and Bolivar, F.: Construction and characterization of new cloning vehicles. VI. Plasmid pBR329, a new derivative of pBR328 lacking the 482-base-pair inverted duplication. Gene 17 (1982) 79-89.]. Plasmid pUC1080 is a derivative of pUC1078 which carries an ~1.9 K.b. BamHI fragment containing a viomycin resistance gene (vph), phenotype designation $Vm^R$, from Streptomyces vinaceus inserted into the BamHI site of pUC1078. These recombinant plasmids can be used for cloning in either E. coli or Streptomyces. Plasmids pUC1072 and pUC1078 are critical intermediates in the construction of pUC1080. Plasmid pUC1080 is a combination promoter-probe-shuttle vector. The antibiotic resistances $Ap^R$, $CM^R$ & $Vm^R$ can be selected in E. coli hosts. The antibiotic resistance $Vm^R$ can be selected in Streptomyces. The single ClaI, BglII and HindIII sites in pUC1080 can be used for cloning. Advantageously, the HindIII site is useful in cloning sequences which can function as operator-promoter regions in Streptomyces by selecting for $Cm^R$ transformants in Streptomyces hosts. Similarly, the HindIII site of pUC1078 alone or in combination with the Bam HI site can be used to clone operator-promoter regions in Streptomyces by selecting $Cm^R$ transformants.

Plasmid pUC1075 is an $Ap^R Cm^R$ plasmid obtained by the covalent linkage of the E. coli plasmid pBR325 to the S. espinosus plasmid pUC1061. Plasmid pUC1079 is a derivative of pUC1075 having an ~1.9 Kb BamHI fragment containing a vph gene from S. vinaceus inserted into the BamHI site of pUC1075. Plasmid pUC1075 is a critical intermediate in the construction of pUC1079. Plasmid pUC1079 is a shuttle vector carrying antibiotic resistances $Ap^R$, $Cm^R$ and $Vm^R$ which are selectable markers in E. coli hosts. The antibiotic resistance $Vm^R$ can be selected in Streptomyces. Plasmid pUC1079 has single sites for endonucleases EcoRI, ClaI, HindIII and BglII which can be used for cloning of DNA sequences. Advantageously, recombinants at the <u>Eco</u>RI site inactivate the <u>cat</u> gene, whereas insertion of DNA into the <u>Cla</u>I or <u>Hind</u>III sites will diminish <u>vph</u> gene expression in <u>E</u>. <u>coli</u>. Full <u>vph</u> gene expression can be detected in <u>Streptomyces</u> hosts. Similarly, the single <u>Eco</u>RI, <u>Cla</u>I, <u>Hind</u>III, <u>Bam</u>HI and <u>Bgl</u>II sites of pUC1075 can be used for cloning of DNA sequences <u>in</u> <u>E</u>. <u>coli</u> hosts. Any gene cloned into pUC1075 can be tested for expression in <u>Streptomyces</u> even though pUC1075 lacks an antibiotic resistance gene known to express in this host.

REFERENCE TO THE DRAWINGS

FIGURE 1 - Restriction endonuclease cleavage map of plasmid pUC1072.

FIGURE 2 - Restriction endonuclease cleavage map for plasmid pUC1078.

FIGURE 3 - Restriction endonuclease cleavage map of plasmid pUC1080.

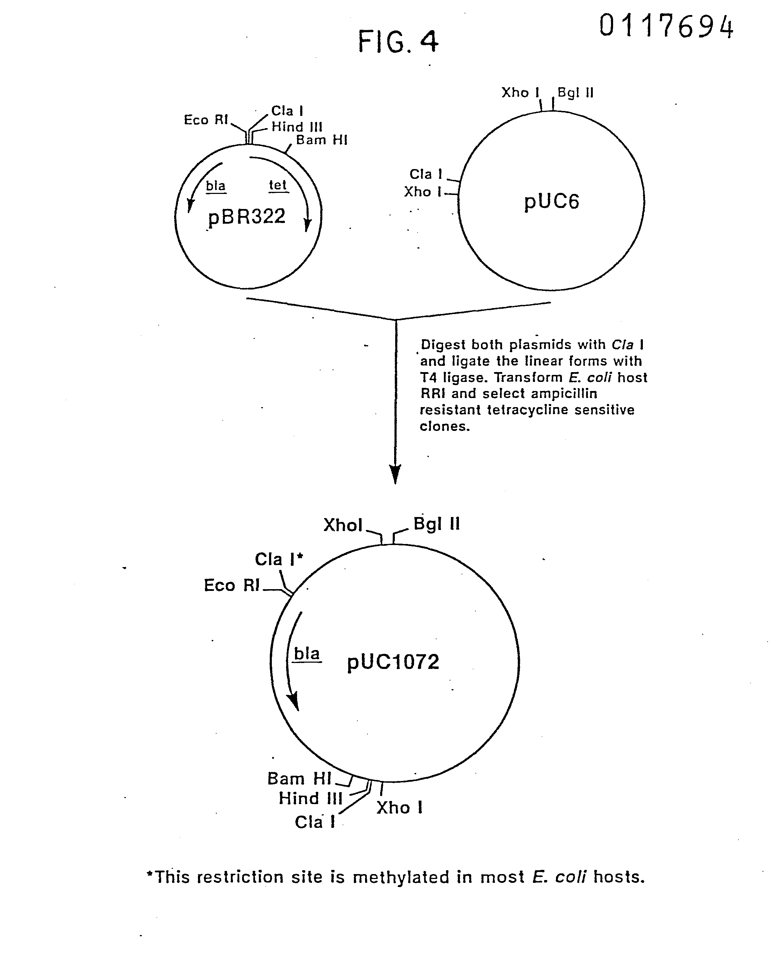

FIGURE 4 - Construction of recombinant plasmid pUC1072.·

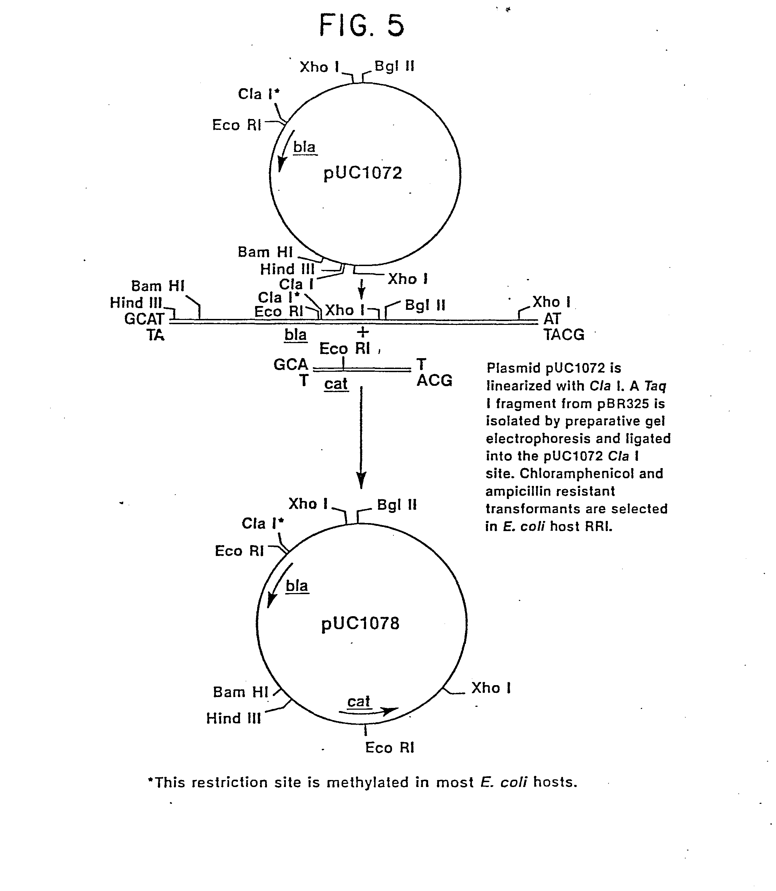

FIGURE 5 - Construction of recombinant plasmid pUC1078.

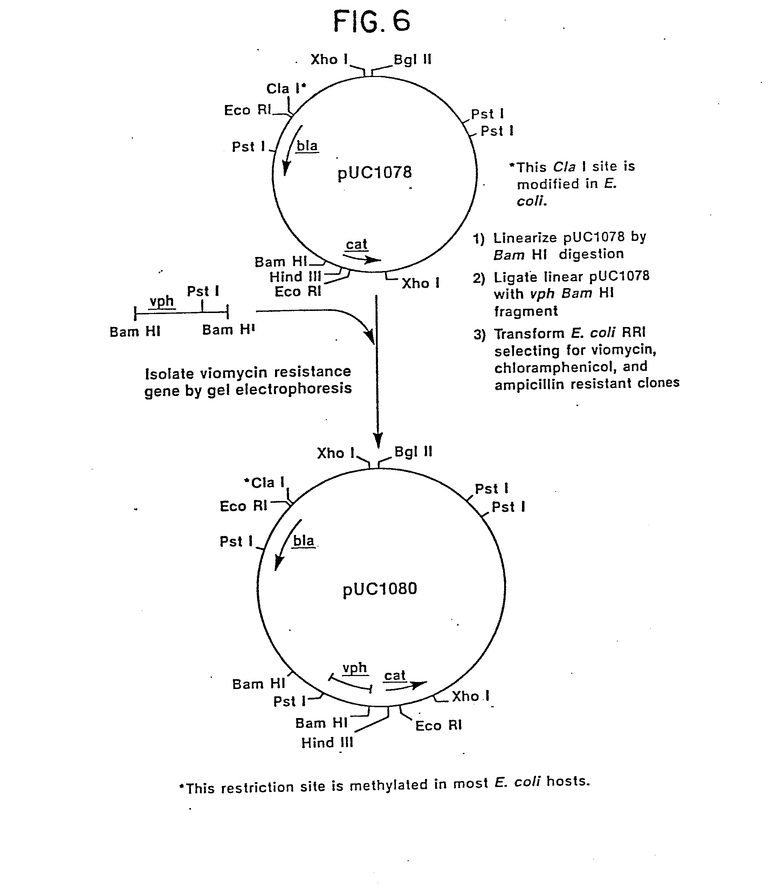

FIGURE 6 - Construction of recombinant plasmid pUC1080.

FIGURE 7 - Restriction endonuclease map of plasmid pUC1075.

FIGURE 8 - Restriction endonuclease map of plasmid pUC1079.

FIGURE 9 - Construction of recombinant plasmid pUC1075.

FIGURE 10- Contruction of recombinant plasmid pUC1079.

The maps are constructed on the basis of plasmids pUC1071, pUC1075, pUC1078, pUC1079 and pUC1080 having molecular weights of <u>ca</u>. 9.0, 8.7, 9.5, 10.1 and 10.8 megadaltons, respectively, or molecular lengths of <u>ca</u>. 13.6, 13.2, 14.4, 15.2 and 16.3 kilobases, respectively. The restriction endonuclease abbreviations are all standard and well known.

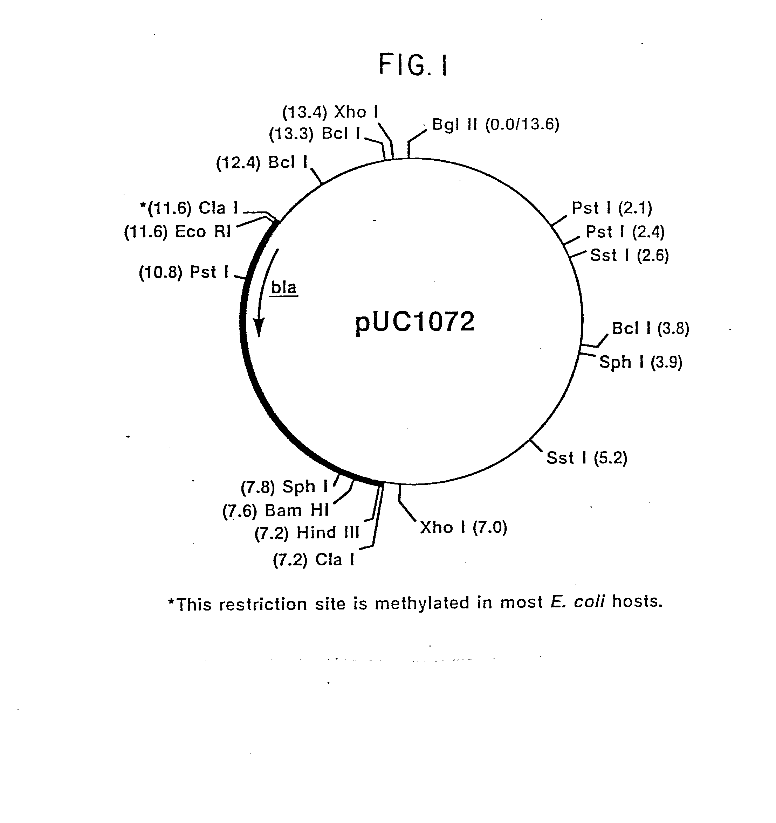

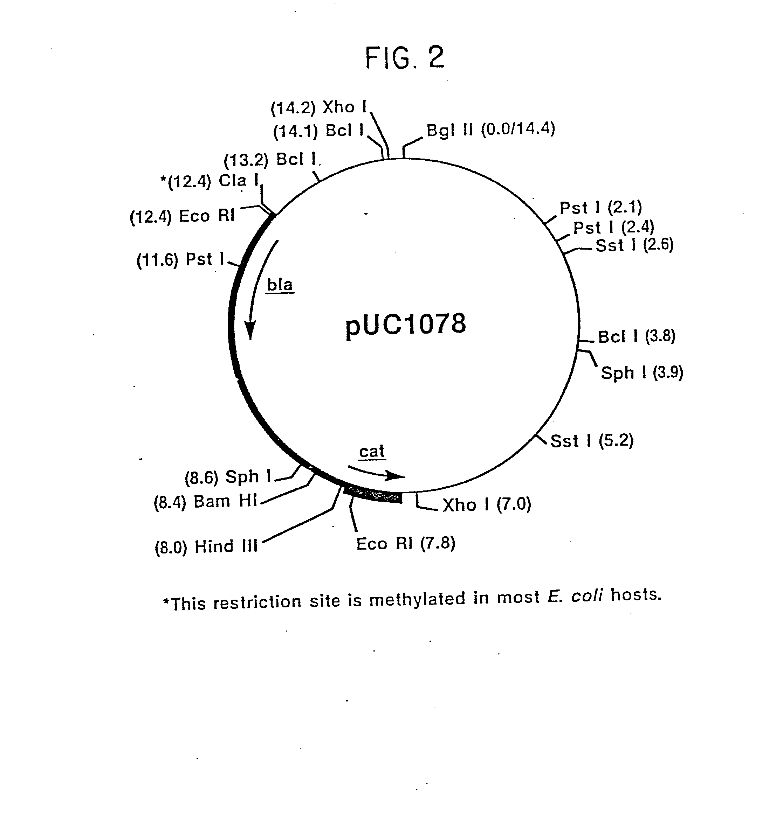

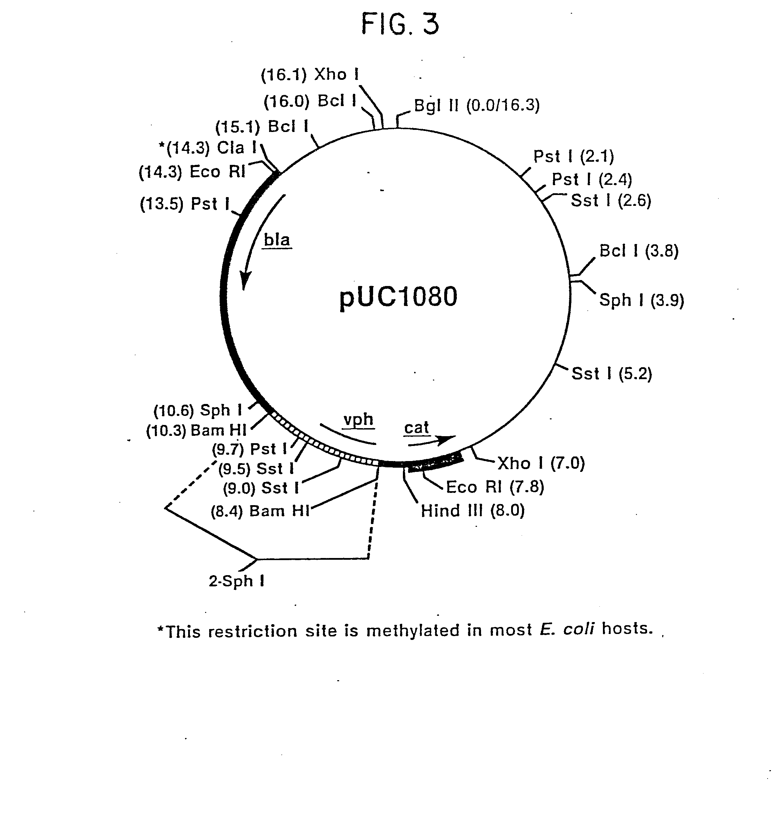

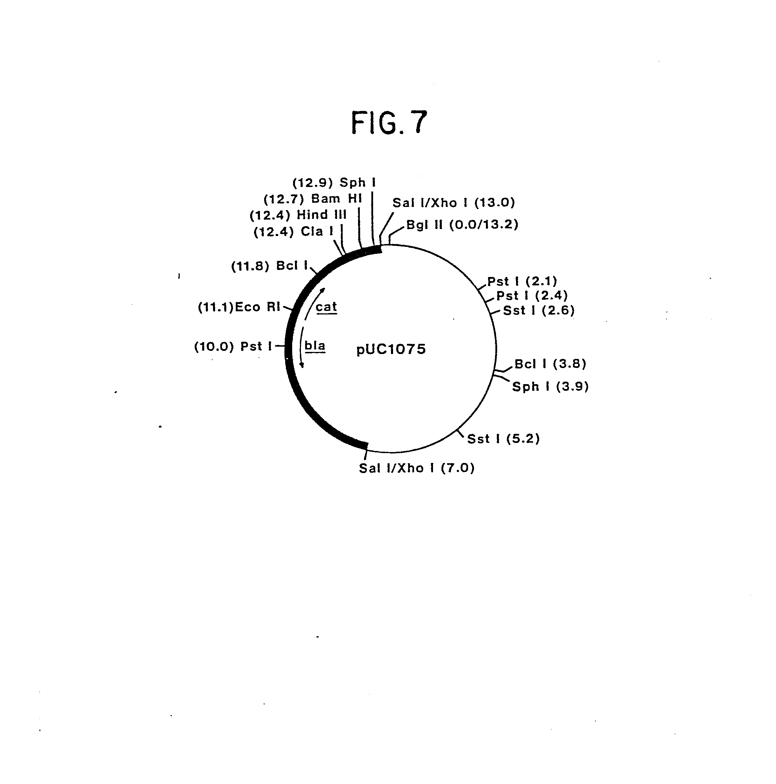

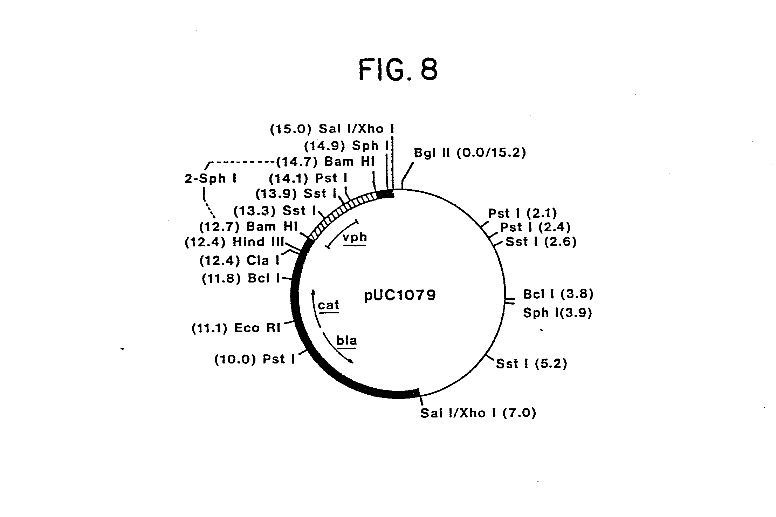

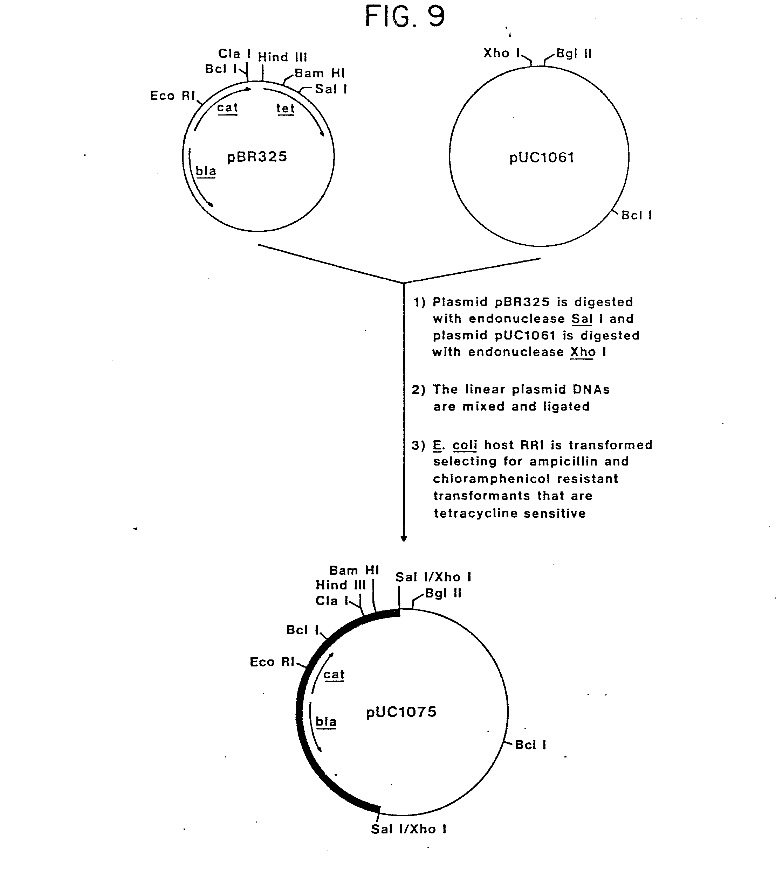

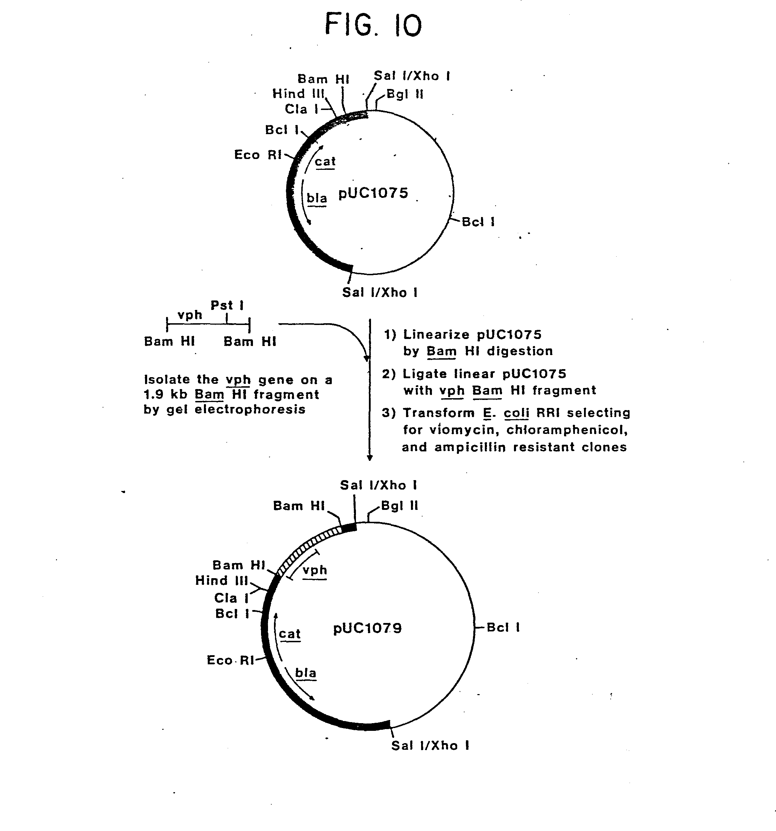

In Figures 1, 2 and 3 the bold lines correspond to pBR322 DNA and the light lines to pUC6 DNA. The doubly bold lines in Figures 2 and 3 correspond to the <u>Taq</u>I fragment from pBR325 carrying the <u>cat</u> gene. In Figures 7-10, the bold lines correspond to pBR325 DNA and the light lines to pUC1061 DNA. In Figures 3, 8 and 10 the segmented line corresponds to the <u>Bam</u>HI fragment carrying the viomycin resistance gene. In all figures the numbers in parentheses correspond to the kilobase coordinates of a particular restriction site relative to the single <u>Bgl</u>II site. The dashed lines bracketing the notation 2-<u>Sph</u>I indicates

that two unmapped SphI sites lie within the viomycin resistance BamHI fragment.

pUC1072, pUC1075, pUC1078, pUC1079 and pUC1080 can be used to create recombinant plasmids which can be introduced into host microbes by transformation. The process of creating recombinant plasmids is well known in the art. Such a process comprises cleaving the isolated vector plasmid at a specific site(s) by means of a restriction endonuclease, for example, BglII, HindIII and the like. The plasmid, which is a circular DNA molecule, is thus converted into a linear DNA molecule or molecular fragments by the enzyme which cuts the two DNA strands at a specific site(s). Other non-vector DNA is similarly cleaved with the same or a different enzyme. Upon mixing the linear vector or portions thereof and non-vector DNAs, their single-stranded or blunt ends can pair with each other and in the presence of a second enzyme known as polynucleotide ligase can be covalently joined to form a single circle of DNA.

The above procedure also can be used to insert a length of DNA from a higher animal into pUC1072, pUC1075, pUC1078, pUC1079 or pUC1080. For example, the DNA which codes for ribosomal RNA in the frog can be mixed with pUC1080 DNA that has been cleaved. The resulting circular DNA molecules consist of plasmid pUC1080 with an inserted length of frog rDNA.

The recombinant plasmids containing a desired genetic element, prepared by using pUC1072, pUC1075, pUC1078, pUC1079 or pUC1080, can be introduced into a host organism for expression. Examples of valuable genes which can be inserted into host organisms by the above described process are genes coding for somatostatin, rat proinsulin, interferon, and proteases.

The usefulness of plasmids pUC1072, pUC1075, pUC1078, pUC1079 and pUC1080 is derived from their capacity to function as plasmid vectors in industrially important microorganisms, e.g., Streptomyces. Also, pUC1072, pUC1075, pUC1078, pUC1079 and pUC1080 are especially useful because of their single restriction sites. Hence, cloning of genetic information from Streptomyces into these plasmids provides a means of increasing the production of commercially important products from these organisms, e.g., antibiotics.

A part of the utility of pUC1072, pUC1075, pUC1078, pUC1079 and pUC1080 is that they consist of DNA sequences from very different bacterial hosts, i.e. hosts that have not been shown capable of exchanging genetic information by normal physiological means. As such, pUC1072, pUC1075, pUC1078, pUC1079 and pUC1080 can function as vectors into both of these organisms (i.e. E. coli and Streptomyces). Plasmids pUC1072, pUC1075, pUC1078, pUC1079 and pUC1080 may also be used as recombinant DNA vectors in conjunction with pUC6 or pUC1061 to transform Streptomyces; whereby, in vivo recombination between pUC6 or pUC1061 and a recombinant derivative of pUC1072, pUC1075, pUC1078, pUC1079 or pUC1080 can give rise to a stable nonchromosomal gene system in the recipient streptomycete.

As a vector, pUC1072 contains single sites for endonucleases BamHI, BglII, EcoRI and HindIII. Though it has two ClaI sites, unexpectedly, one of these (i.e., at 11.6 kb) is blocked by methylation of an adenine nucleotide in DNA adenine methylase proficient (dam$^+$) E. coli hosts. In this manner one, advantageously, can obtain a pUC1072 molecule having one cleavable ClaI site. Plasmid pUC1072 can readily be obtained from various E. coli hosts. Plasmid pUC1072 has a molecular weight of ~ 9.0 x 10$^6$ daltons. By comparison, plasmid pUC1078 has a molecular weight of ~ 9.5 x 10$^6$ daltons and has single cleavage sites for endonucleases BamHI, BglII, HindIII and ClaI. The ClaI site is available for cloning only in dam$^-$ hosts. Plasmid pUC1078 carries a CM$^R$ gene in addition to the Ap$^R$ gene of pUC1072. Because of its construction, pUC1078 can be used to clone DNA sequences which can function as operator-promoter regions in Streptomyces. This can be accomplished by the insertion of DNA at the HindIII site of pUC1078 followed by selection of CM$^R$ transformants in Streptomyces. Plasmid pUC1080 has single sites for endonucleases HindIII, BglII and ClaI. The ClaI site is available for cleavage only DNA propagated in dam$^-$ hosts. Plasmid pUC1080 has a molecular weight of ~ 10.8 x 10$^6$ daltons and can be used like pUC1078 for cloning of DNA sequences at the HindIII that will function as operator-promoter regions in Streptomyces. pUC1080 has the advantage that it also carries a gene for viomycin resistance which expresses in either E. coli or Streptomyces. This allows selection of transformants in Streptomyces in the absence of easily detectable phenotypes conferred by cloned DNA sequences.

Like pUC1072, pUC1075 contains single sites for endonucleases BamHI, BglII, EcoRI and HindIII. Unlike pUC1072, pUC1075 contains a

single ClaI site which is not blocked by methylation in dam[+] hosts. Consequently, the plasmid, advantageously, has a single ClaI site for cloning in either dam[+] or dam[-] hosts. Plasmid pUC1075 can readily be obtained from various E. coli hosts and it has a molecular weight of ~10.1 x $10^6$ daltons. By comparison, plasmid pUC1079 has a molecular weight of ~10.1 x $10^6$ daltons and has cleavage sites for EcoRI, ClaI, HindIII and BglII. As with pUC1075, the pUC1079 ClaI site is available for cleavage in both dam[+] and dam[-] hosts. Insertion of foreign DNA into the EcoRI site of either pUC1075 or pUC1079 disrupts the Cm[R] gene. Insertion of foreign DNA into the ClaI or HindIII sites of pUC1079 diminishes expression of viomycin resistance. Hence, plasmids pUC1075 and pUC1079 are uniquely suited vectors and can readily be used for cloning DNA sequences which do not express or express poorly in E. coli by looking for insertional inactivation of genes or increased sensitivity to viomycin. Expression of such cloned genes can then be tested in Streptomyces.

In plasmids pUC1072, pUC1075, pUC1078, pUC1079 and pUC1080 the ClaI sites can be used to clone DNA cleaved by endonucleases ClaI, TaqI, HpaII, AccI, AcyI, AsuII. The BamHI and/or BglII sites of the plasmids are compatible with cloning BamHI, BglII, BclI, MboI or Sau3A digested DNA. Any of the single sites can be modified by means well known in the art to accept either blunted DNAs or DNAs that have been tailed with synthetic linkers.

This approach is compared to the concept of cloning genes for antibiotic production into the well characterized Escherichia coli K-12 host-vector system. The E. coli system has the disadvantage that it has been found that genes from some Gram-positive organisms, e.g., Bacillus, do not express well in the Gram-negative E. coli host. Similarly, reports have appeared that indicate Streptomyces DNA is not expressed in E. coli without coupling to E. coli regulatory sequences. Likewise, plasmids from Gram-negative organisms are not maintained in Gram-positive hosts, and Gram-negative genetic information is either expressed poorly or not at all in Gram-positive hosts. This clearly argues for the advantage of a Gram-positive host-vector system and argues the usefulness of plasmids pUC1072, pUC1075, pUC1078, pUC1079 or pUC1080 in such a system. Because pUC1072, pUC1075, pUC1078, pUC1079 and pUC1080 are also capable of replication and maintenance in E. coli, they are desirable vectors for those situations where cloning

0117694

4062.1

a gene(s) in E. coli is a desirable first step before transferring that gene to say Streptomyces.

In general, the use of a host-vector system to produce a product foreign to that host requires the introduction of the genes for the entire biosynthetic pathway of the product to the new host. As discussed above, this may lead to problems of genetic expression, but may also generate new and/or increased problems in the fermentation of the microorganisms and in the extraction and purification of the product. A perhaps more useful approach is to introduce a plasmid vector into a host which normally produces the product and clone onto that plasmid the genes for biosynthesis of the product. At the very least, problems of fermentation and product extraction and purification should be minimized. Additionally, in this cloning system it may not be necessary to clone and amplify all the genes of the biosynthetic pathway, but rather it may be necessary only to clone regulatory genes or genes coding for the enzymes that are rate limiting in product biosynthesis. Since pUC1072, pUC1075, pUC1078, pUC1079 and pUC1080 are recombinant plasmids which contain DNA from organisms that do not normally exchange genetic information, they can be used to clone DNA sequences in E. coli or within the genera of Streptomyces and Micromonospora, as well as within other microbes.

DETAILED DESCRIPTION OF THE INVENTION

The Microorganisms and Plasmids

The following microorganisms are available from the permanent collection of the Northern Regional Research Laboratory, U.S. Department of Agriculture, Peoria, Illinois, U.S.A.

| | | | Deposited |
|---|---|---|---|
| (1) | NRRL B-12014 | - E. coli RR1 (pBR322) | Known and available before the filing date of the subject application |
| (2) | NRRL B-12186 | - E. coli RR1 | Known and available before the filing date of the subject application |
| (3) | NRRL 15276 | - S. lividans 1326 | February 4, 1983 |
| (4) | NRRL 15277 | - S. lividans 1326 (pUC1080) | February 4, 1983 |

(5)  NRRL B-15278  -  E. coli RR1 (pUC1072)    February 7, 1983

(6)  NRRL B-15279  -  E. coli RR1 (pUC1078)    February 7, 1983

(7)  NRRL B-15280  -  E. coli RR1 (pUC1080)    February 7, 1983

(8)  NRRL B-15295  -  E. coli RR1 (pBR325)     February 16, 1983

(9)  NRRL 11439    -  S. espinosus biotype
     23724a        -                           Known and available
                                               before the filing date
                                               of the subject applica-
                                               tion

(10) NRRL 12488    -  S. espinosus biotype
     23724a        -  (pUC1061)                Known and available
                                               before the filing date
                                               of the subject applica-
                                               tion

(11) NRRL 15528    -  S. lividans 1326         July 18, 1983
                      (pUC1079)

(12) NRRL B-15527  -  E. coli RRI              July 19, 1983
                      (pUC1075)

(13) NRRL B-15526  -  E. coli RR1              July 19, 1983
                      (pUC1079)

These deposits are available to the public upon the grant of a patent to the assignee, The Upjohn Company, disclosing them. The deposits are also available as required by foreign patent laws in countries wherein counterparts of the subject application, or its progeny, are filed. However, it should be understood that the availability of a deposit does not constitute a license to practice the subject invention in derogation of patent rights granted by governmental action.

The following examples are illustrative of the process and products of the subject invention but are not to be construed as limiting. All percentages are by weight and all solvent mixture proportions are by volume unless otherwise noted.

Example 1    Isolation of Vector pBR322 or pBR325 DNA from E. coli NRRL
             B-12014 or E. coli NRRL B-15295, respectively

A 100 ml. culture of E. coli RR1 (pBR322) or E. coli RRI (pBR325) is grown overnight in L-broth which consists of the following ingredients:

    Bacto tryptone (Difco)              10 g./liter
    Bacto yeast extract (Difco)          5 g./liter

NaCl                                      5 g./liter

Ampicillin                                50 mg./liter

The cells are recovered by centrifugation at 17,000 x g. for 10 minutes in a refrigerated centrifuge. The pellet is suspended in 2.5 ml. 50 mM Tris buffer (pH 8) containing 25% sucrose. One-half ml. of lysozyme stock solution is added (5 mg./ml. in TES buffer). The mixture is allowed to stand in ice for 5 minutes. At this point 1 ml. EDTA (0.25 M, pH 8) is added and the mixture is again allowed to stand in ice for 5 minutes. One and a quarter ml. of 5 M NaCl and 1 ml. 10% SDS (sodium dodecyl sulfate) are then added. The mixture is shaken on a Vortex and incubated at 37°C. for 20 minutes. Then 10 μl of ribonuclease (20 mg./ml.) is added and the sample is again incubated at 37°C. for 20 minutes. The mixture is then kept in ice overnight and then centrifuged at 35,000 x g. for 30 minutes in a refrigerated centrifuge. 2 ml. of the supernatant solution (lysate) are carefully removed with a pipette. Four and one-half ml. of TES buffer (30 mM Tris·HCl, pH 8, 5 mM EDTA·Na$_2$, 50 mM NaCl) are mixed with 1.5 ml. EtBr (ethidium bromide) stock (1 mg./ml. in TES buffer) and 7.5 g. solid CsCl. After the salt has dissolved, 2 ml. of the lysate, described above, is added and the mixture is transferred into a polyallomer tube fitting a titanium 50 (50 Ti) head (Beckman ultracentrifuge). The tubes are filled to the top with mineral oil and centrifuged in a Beckman ultracentrifuge at 40,000 rpm in a 50 Ti head at 15°C. for at least 2 days. The DNA is located under a long wave UV-lamp and the heavier band containing the plasmid DNA is removed with a syringe by puncturing the tube wall from the side. The samples are extensively dialyzed against 200 volumes of TES buffer at 4°C. Following dialysis, 1/10 sample volume of a 3 M NaAcetate stock solution is added and the plasmid DNA is precipitated by the addition of 2 volumes of cold ethanol. The resulting pellet is then lyophilized and redissolved in 200 μl 10 mM Tris buffer, pH 7.8 containing 1 mM EDTA·Na$_2$ and frozen for storage.

Example 2 - Isolation of Plasmid pUC6 or pUC1061 DNA from Biologically Pure Cultures of Streptomyces espinosus, biotype 23724a, NRRL 11439 or NRRL 12488, respectively

The spores from a biologically pure culture of Streptomyces espinosus biotype 23724a, NRRL 11439 (pUC6) or NRRL 12488 (pUC1061) are inoculated into 10 ml of the following Difco Antibiotic Medium No.

3 Broth (Difco Labs., Detroit, Michigan): 0.15% Beef extract; 0.15% yeast extract; 0.5% peptone; 0.1% glucose; 0.35% $\underline{\text{Na}}$Cl; 0.368% $K_2HPO_4$; 0.132% $KH_2PO_4$.

The medium has previously been sterilized in a 50 ml. Erlenmeyer flask. After inoculation, the flask is incubated at 37°C. for about 36 to 48 hours on a Gump or New Brunswick rotary shaker operating at 100-250 rpm. Upon completion of the incubation, the mycelia-broth suspension in the flasks is homogenized under sterile conditions and is then mixed in a sterile 125 ml. Erlenmeyer flask containing 10 ml. of the above medium and also, advantageously 68% (w/v) sucrose and 1% (w/v) glycine. The addition of sucrose and glycine facilitates the subsequent lysing of the cells. The amounts of sucrose and glycine in the medium can be varied by routine adjustments with the goal being to facilitate the subsequent lysing of the cells. The flask is then incubated further for another 36 to 48 hours at 37°C. on a Gump rotary shaker, as above. After this incubation, the mycelia are separated from the broth by low speed centrifugation, for example, at 6000 x g. for 15 minutes at 4°C. and decantation of the supernatant from the mycelial pellet.

The supernatant is discarded and the pellet is resuspended in 1.5 ml. of an isotonic buffer containing ethylenediaminotetraacetic acid (EDTA) and sucrose, e.g., TES buffer [0.03 $\underline{M}$ tris(hydroxymethyl)amino-methane (Tris), 0.005 $\underline{M}$ EDTA and 0.05 $\underline{M}$ NaCl; pH = 8.0] containing 20% (w/v) sucrose. Next, 1.5 ml. of a 5 mg./ml. solution of lysozyme in the same buffer is added and the mixture is incubated at 37°C. for 30 minutes with occasional mixing. Then, 1.5 ml. of 0.25 $\underline{M}$ EDTA (pH = 8.0) is added and this mixture is incubated 15 minutes at 37°C. Subsequently, the cell suspension is lysed by the addition of 2.5 ml. of a lytic mixture, e.g. [1.0% (w/v) Brij-58 (a detergent sold by Pierce Chem. Co., Rockford, Illinois), 0.4% (w/v) deoxycholic acid, 0.05 $\underline{M}$ Tris (pH = 8.0) and 0.06 $\underline{M}$ EDTA] and incubation of this mixture at 37°C. for 20 minutes. The lysate is then sheared by passing it 5-10 times through a 10 ml. pipette. The sheared lysate is then digested with ribonuclease (140 µg/ml.) and pronase (300 µg/ml.) for an additional 20 minutes at 37°C. Alternatively, the cell-lysozyme-EDTA mixture can be digested with ribonuclease and pronase before lysis with a lytic agent such as 2% sodium dodecyl sulfate in water.

This crude lysate material is then mixed with a salt, for example, cesium chloride (preferred), and cesium sulfate, and the intercalating dye ethidium bromide to give a solution of density $\rho$ = 1.550. This solution is centrifuged to equilibrium at 145,000 x g. (isopycnic density gradient centrifugation). The covalently closed circular plasmid DNA is then visible in the centrifuge tube under long wave ultraviolet (320 nm) illumination as a faint fluorescent band below the intensely fluoresent band of linear chromosomal and plasmid DNA's.

Covalently closed circular plasmid DNA is prepared for characterization by removing it from the isopycnic gradients, extracting the ethidium bromide by two treatments with one-third volume of isopropyl alcohol and then dialyzing the aqueous phase against an appropriate buffer, e.g., 0.1 x SSC buffer (0.015 $\underline{M}$ NaCl, 0.0015 $\underline{M}$ sodium citrate; pH = 7.4) to yield essentially pure pUC6 or pUC1061.

Characteristics of pUC6 and pUC1061

These are disclosed in U.S. Patent 4,273,875, and are published [Manis, J.J. and Highlander, S.K.: Partial characterization of a small, multi-copy plasmid from Streptomyces espinosus and the derivation of a high copy-number deletion mutant. Gene 18 (1982) 13-20.].

Characteristics of pBR322

These are published [Sutcliffe, J.G.: Complete nucleotide sequence of the Escherichia coli plasmid pBR322. In Cold Spring Harbor Symposia on Quantitative Biology. Vol. 43, pg 77-90, Cold Spring Harbor Laboratory, New York].

Characteristics of pBR325

Plasmid pBR325 has been described [Bolivar, F.: Construction and characterization of new cloning vehicles III. Derivatives of plasmid pBR322 carrying unique EcoRI sites for selection of EcoRI generated recombinant DNA molecules. Gene 4 (1978) 121-136 and Prentki, P., Karch, F., Iida, S. and Meyer, J.: The plasmid cloning vector pBR325 contains a 482 base-pair-long inverted duplication. Gene 14 (1981) 289-299].

Example 3      Preparation of Recombinant Plasmid pUC1072 and Transformation into E. coli RR1.

Plasmids pUC6 and pBR322, prepared as described above, are linearized by digestion with restriction endonucleases ClaI. Plasmid DNAs are digested with ClaI restriction enzyme by mixing ~ 50 µl. of DNA (~ 0.5 µg) solution in TE buffer (0.01 $\underline{M}$ Tris •HCl, 0.001 $\underline{M}$ Na$_2$

EDTA; pH 8.0) with 50 µl of 2X restriction buffer (20mM Tris-HCl, pH 8.0; 20mM $MgCl_2$) and 4 units of ClaI enzyme preparation. This mixture is incubated at 37°C. for 1 hour. The digest is then applied to a 1% preparative low melting point agarose gel and electrophoresed for ~ 3 hours at 50 volts and 4°C. The resolved DNA fragments are visualized by ethidium bromide staining and long wave ultraviolet light illumination. The region of the gel containing the DNA is excised from gel and heated to 65°C. in the presence of 1.5 ml. of TE buffer to melt the gel and release the DNA from the gel matrix. This suspension is chilled and centrifuged at 37,000 xg to pellet the agarose. The supernatant is decanted and saved. The agarose pellet is extracted a second time with TE buffer. The two supernatants are pooled and ethanol precipitated by the addition of 0.1 volume of 3 M Na Acetate and 2 volumes 95% ethanol at -20°C. The DNA precipitate is collected by centrifugation at 85,000 xg at 4°C. for 60 minutes. The precipitate is redissolved in 100 µl of TE buffer. This sample is used for ligation as described below.

For ligation, 25 µl of ClaI digested pBR322 DNA, 25 µl of ClaI digested pUC6 DNA and 20 µl DD $H_2O$ are combined. Ten µl 100 mM DDT, 10 µl 50 mM $MgCl_2$ and 10 µl of 0.5 mM ATP are combined with the restricted DNA mixture. Finally, 1.0 u of $T_4$ DNA ligase is added and the sample is kept in ice for 1-2 days or incubated 1-2 hrs at room temperature (~22° C).

The ligated sample is. ethanol precipitated and used to transform E. coli culture RR1 to ampicillin resistance ($Ap^r$).

For transformation into E. coli RR1, an inoculum is grown overnight in L-broth and diluted 1:100 into fresh L-broth the next day. The cells are incubated at 37°C. and allowed to grow to an $OD_{650}$ of 0.2. At this point 50 ml. of culture is centrifuged in the cold, the pellet resuspended in 20 ml. cold 100 mM $CaCl_2$, incubated at 0°C. for 20-25 minutes and centrifuged again. The pellet is then resuspended in 0.5 ml. cold 100 mM $CaCl_2$ solution and kept at 0-4°C. for 24 hours. (Dagert, M. and Ehrlich, S. D. 1979, Gene 6: 23-28) One hundred µl of ligation mixture (see above) is mixed with 500 µl cell suspension. This mixture is kept in ice for 10 minutes, and then at 37°C. for 5 minutes. Ten to 20 ml. of L-broth is added and the cell suspension is incubated 1-2 hours at 37°C. Next, 100 µl aliquots are plated on freshly prepared agar plates containing 25 ml. of L-broth, 1.5% agar, and 50 µg of ampicillin/ml.  Colonies are selected and scored for tetracycline sensitivity.

Suspected recombinant DNA containing transformants are then grown in 25 ml. cultures.  Cleared lysates are prepared by pelleting the cells from the culture medium at ~ 10,000 xg.  The pellet is resuspended in 10 ml. of cold TES buffer (30 mM Tris·HCl, 5 mM Na$_2$ EDTA and 50 mM NaCl, pH 8.0) and pelleted again.  This pellet is resuspended in 1 ml. of TES buffer containing 20% sucrose.  0.2 ml. of lysozyme solution (5 mg./ml. in TES) is added and incubated on ice 15 minutes at which time 0.4 ml. of 0.25 M Na$_2$ EDTA (pH 8.0) is added and the incubation continued 15 minutes.  1.6 ml. of a lytic mix (1% Brij 58, 0.4% Na deoxycholate, 0.05 M Tris·HCl, 62.5 mM Na$_2$ EDTA; pH 8.0) is added and the lysate incubated an additional 15 minutes at 4°C.  The lysate is sheared by passage 5 times through a 10 ml. pipette.  The bulk of the cellular DNA and debris are removed by centrifugation at 48,000 xg for 30 minutes.  The cleared lysate is digested successively for 15-minute intervals with pancreatic RNAse A (100 mcg./ml.) and Pronase (200 mcg./ml.) at 37°C.  These lysates are then centrifuged in CsCl-ethidium bromide isopycnic density gradients.  Plasmid DNA isolated from these gradients is characterized by digestion with restriction endonucleases.

Example 4        Isolation of Plasmid pUC1072 From NRRL B-15278- E. coli RR1 (pUC1072)

Plasmid pUC1072 can be isolated from its E. coli host by well known procedures, e.g., using the cleared lysate-isopycnic density gradient procedures.  Once transformants containing pUC1072 are identified, they are separated as pure entities in a pure culture.  This plasmid can be differentiated as a distinct entity by its unique restriction patterns as would be predicted by its restriction map (Fig. 1).  Note:  When plasmid pUC1072 is maintained in a DNA modification proficient host such as E. coli RR1, the BclI restriction sites and the ClaI site at 11.6 kb  are modified such that they can not be detected by endonuclease digestion.  However, maintenance of pUC1072 in an adenine methylase deficient host allows the demonstration of the presence of BclI and the 11.6 kb ClaI restriction sites.

Example 5        Preparation of Recombinant Plasmid pUC1078 and Transformation into E. coli RR1.

Plasmid pUC1072 can be obtained as described in Example 4.  The pUC1072 DNA is then digested with ClaI using the procedures disclosed in Example 3. The resulting linear DNA fragment is purified by agarose gel electrophoresis, also as described in Example 3. Similarly, pBR325, a chloramphenicol resistant derivative of pBR322, can be isolated as described in Example 2 from an E. coli host. This DNA is subjected to digestion by endonuclease TaqI. The third largest TaqI fragment is isolated from a preparative agarose gel as described in Example 3. This fragment is mixed with linear ClaI digested pUC1072 DNA, ligated with T4 DNA ligase and used to transform E. coli RR1 to ampicillin resistance. These procedures also are as described in Example 4.

Recombinant plasmids were characterized by cleavage with restriction endonucleases. Restriction endonucleases were obtained as commercial preparations from Miles Laboratories, Bethesda Research Laboratories and New England Biolabs. Enzyme digestions were prepared in accordance with the conditions specified by the suppliers using at least a two-fold excess of endonuclease. The digested samples were applied to 0.7-1% agarose gels and were electrophoresed for 2 hours at a constant applied voltage of 10-15 v/cm of gel height. [Sharp, P. A., Sugden, J. and Sambrook, J. 1973. Detection of two restriction endonuclease activities in Haemophilus parainfluenzae using analytical agarose-ethidium bromide electrophoresis. Biochemistry 12, 3055-3063]. The molecular weights of restriction fragments were determined relative to the standard migration patterns of bacteriophage lambda DNA digested with enzyme HindIII [Murray, K. and Murray, N. E. 1975. "Phage lambda receptor chromosomes for DNA fragments made with restriction endonuclease III of Haemophilus influenzae and restriction endonuclease I of Escherichia coli." J. Mol. Biol. 98, 551-564] or EcoRI [Helling, R. B., Goodman, H. M. and Boyer, H. W. 1974. "Analysis of endonuclease R·EcoRI fragments of DNA from lambdoid bacteriophages and other viruses by agarose-gel electrophoresis." J. Virology 14, 1235-1244].

Example 6        Preparation of Recombinant Plasmid pUC1080 and
                 Transformation into E. coli RR1 and S. lividans 1326.

Recombinant plasmid pUC1078 can be prepared from E. coli cultures in a manner analogous to that described for preparation of plasmid pUC1072 in Example 4. Plasmid pUC1078 is linearized by digestion with endonuclease BamHI and the linear fragment isolated by preparative agarose gel electrophoresis as described in Example 3. S. vinaceus chromosomal DNA can be isolated by means well known in the art. S. vinaceus is disclosed in U.S. Patent 2,633,445 with the accession number NRRL 2285. It is also available as NCIB 8852 from the National Collection of Industrial Bacteria, Aberdeen, Scotland. Chromosomal DNA is digested with endonuclease BamHI and the fragments separated by preparative agarose gel electrophoresis. The DNA banding at 1.9 kb on the gel is extracted as described in Example 3. A viomycin resistance gene (vph) is carried on a 1.9 kb BamHI fragment from S. vinaceus chromosomal DNA [Thompson, C.J., Skinner, R.H., Thompson, J., Ward, J.M. Hopwood, D.A. and Cundlitte: Biochemical characterization of resistance determinants cloned from antibiotic-producing streptomycetes. J. Bacteriol. 151 (1982) 678-685].

The 1.9 kb fragments and the BamHI linearized puC1078 DNA are mixed together (~0.5 µg of each is used), ligated and transformed into E. coli RR1 as described in Example 3. Transformants are selected from $Cm^R Vm^R$ (resistance to chloramphenicol and viomycin, respectively) on media containing 20 µg/ml chloramphenicol and 1000 µg/ml viomycin. These are subsequently tested for ampicillin resistance and tetracycline sensitivity. From this transformation plasmid pUC1080 (Fig. 3) was isolated. pUC1080 was characterized in a manner similar to pUC1078 described in Example 5.

Plasmid pUC1080 can be isolated from E. coli hosts in a manner analogous to pUC1072 as described in Example 4. This DNA can be used to transform Streptomyces cultures in the following manner. S. lividans culture 1326 NRRL 15276 is inoculated from a spore preparation into 10 ml of S-medium [Okanishi, M., Suzuki, K. and Umezawa, H.: Formation and reversion of streptomycete protoplasts: cultural condition and morphological study. J. Gen. Microbiol. 80 (1974) 389-400)] and grown 24-36 hrs at 28° C on a rotary shaker. The suspension is ground in a sterile homogenizer and brought to 0.5% glycine by addition of an equal volume of S-medium containing 1.0% glycine. The incubation is continued for 18-24 hrs at 28° C with shaking. The mycelia are harvested by centrifugation and washed with 5 ml of sterile 0.3 M sucrose. The pellet is resuspended in 5 ml of 0.3 M sucrose and sonicated in a Branson sonic water bath for 15-30 min to break up the mycelia. This suspension is pelleted and resuspended in 5 ml of sterile medium P [Okanishi, M., Suzuki, K and Umezawa, H.:

Formation and reversion of streptomycete protoplasts: cultural condition and morphological study. J. Gen. Microbiol. 80 (1974) 389-400] containing 5 mg/ml lysozyme. This suspension is incubated at 37° C for 1-4 hrs to form protoplasts. The protoplasts are separated from the mycelia by decanting and filtration through compressed cotton. The filtered protoplasts are pelleted at ~ 2000 g in a clinical centrifuge and washed twice with sterile medium P. Finally, the protoplasts are resuspended in 1.0 ml of medium P. A sample of pUC1080 DNA in TE buffer (~1.0 μg in 0.1 ml) is added followed immediately by the addition of 1.0 ml of medium P containing 20% w/v polyethylene glycol (PEG) 6000. This is mixed quickly and allowed to incubate 1-5 min at room temperature. Five ml of medium P is then added and the protoplasts are pelleted, resuspended in 1.0 ml of medium P and plated onto R2 medium [Okanishi, M., Suzuki, K. and Vmezawa, H.: Formation and reversion of streptomycete protoplasts: cultural condition and morphological study. J. Gen. Microbiol. 80 (1974) 389-400] using an R2 soft agar medium overlay. After the protoplasts have generated and formed spores at 28° C, the spores are collected and plated onto Hickey-Tresner agar containing 100-200 μg/ml viomycin. The Vm$^R$ clones are checked for plasmid DNA as described in Example 3 and the plasmid is characterized as described in Example 5.

Example 7 -    Preparation of Recombinant Plasmid pUC1075 and Transformation into E. coli RR1

Plasmids pUC1061 and pBR325, prepared as described in Examples 1 and 2, are linearized by digestion with restriction endonucleases XhoI and SalI, respectively. Plasmid DNAs are digested with XhoI by mixing 50 μl of DNA (~0.5 μg) solution in TE buffer with 50 μl of 2x restriction buffer (16mM Tris-HCl, pH 7.4; 12 mM MgCl$_2$; 300 mM NaCl; 12 mM 2-mercuptoethanol) and 4 units of XhoI enzyme preparation. Endonuclease SalI digests are done in a similar manner and with identical buffer systems except that the 2x restriction buffer is pH 7.9. The resulting linear DNAs are purified by agarose gel electrophoresis as described in Example 3. The purified linear plasmids are mixed, ligated with T4 DNA ligase and used to transform E. coli RR1. These procedures are also described in Example 4. Transformants exhibiting Ap$^R$Cm$^R$ and Tc$^S$ (tetracycline sensitivity) are selected by plating on medium containing the appropriate antibiotics. It is possible to isolate SalI:XhoI recombinants of pUC1061 and pBR325 in this manner because the single-stranded ends of produce by cleavage of DNA with these two endonucleases will base pair and allow ligation to occur.

Recombinant plasmids were isolated from various transformants as described in Examples 3 and 4. They were characterized by endonuclease digestion as described in Example 5 to insure the correct construction and to determine the orientation of the two plasmids with respect to one another.

Plasmid pUC1075 was obtained from several transformants. pUC1075 can be obtained in pure form from culture NRRL B-15527 by the procedures described in Example 4.

Example 8 -    Preparation of Recombinant Plasmid pUC1079 and Transformation into E. coli RR1 and S. lividans 1326

Recombinant plasmid pUC1079 can be prepared from E. coli cultures in a manner analogous to that described for preparation of plasmid pUC1072 in Example 4. Plasmid pUC1075 is linearized by digestion with endonuclease BamHI and the linear fragment isolated by preparative agarose gel electrophoresis as described in Example 3. S. vinaceus chromosomal DNA can be isolated by means well known in the art. S. vinaceus is disclosed in U.S. Patent 2,633,445 with the accession number NRRL 2285. It is also available as NCIB 8852 from the National Collection of Industrial Bacteria, Aberdeen, Scotland. That chromosomal DNA is digested with endonuclease BamHI and the fragments separated by preparative agarose gel electrophoresis. The DNA banding at 1.9 kb on the gel is extracted as described in Example 3. A viomycin resistance gene (vph) is carried on a 1.9 kb BamHI fragment from S. vinaceus chromosomal DNA [Thompson, C.J., Skinner, R.H., Thompson, J., Ward, J.M., Hopwood, D.A. and Cundlitte: Biochemical characterization of resistance determinants cloned from antibiotic-producing streptomycetes. J. Bacteriol. 151 (1982) 678-685].

The 1.9 kb fragments and the BamHI linearized pU1075 DNA are mixed together (~0.5 µg of each is used), ligated and transformed into E. coli RR1 as described in Example 3. Transformants are selected for $Ap^R Cm^R Vm^R$ (resistance to ampicillin, chloramphenicol and viomycin, respectively) on media containing 20 µg/ml chloramphenicol and 1000 µg/ml viomycin. These are subsequently tested for tetracycline sensitivity. From this transformation plasmid pUC1079 (Fig. 3) was isolated. pUC1079 was characterized in a manner similar to pUC1078 described in Example 5.

Plasmid pUC1079 can be isolated from E. coli hosts in a manner analogous to pUC1072 as described in Example 4. This DNA can be used to transform Streptomyces cultures by the procedures described in Example 6.

Examples of other hosts for the vectors are any E. coli K-12 derivative [Bacteriological Reviews, Dec. 1972, pages 525-557] (these are listed as approved hosts in the NIH Guidelines) and yeasts, other fungi, or other bacteria. It is recognized that these latter hosts would also have to fill the criteria for approved hosts as described in the NIH Guidelines.

## CLAIMS

1.    E. coli RR1 (pUC1072) having the deposit accession number NRRL B-15278.

2.    E. coli RR1 (pUC1078) having the deposit accession number NRRL B-15279.

3.    E. coli RR1 (pUC1080) having the deposit accession number NRRL B-15280.

4.    Recombinant plasmid pUC1072, characterized as shown by the restriction map in Figure 1 of the drawings.

5.    Recombinant plasmid pUC1078 characterized as shown by the restriction map in Figure 2 of the drawings.

6.    Recombinant plasmid pUC1080 characterized as shown by the restriction map in Figure 3 of the drawings.

7.    E. coli RR1 (pUC1075) having deposit accession number NRRL B-15527.

8.    E. coli RR1 (pUC1079) having deposit accession number NRRL B-15526.

9.    Recombinant plasmid pUC1075, characterized as shown by the restriction map in Figure 7 of the drawings.

10.   Recombinant plasmid pUC1079, characterized as shown by the restriction map in Figure 8 of the drawings.

# FIG. I

*This restriction site is methylated in most *E. coli* hosts.

# FIG. 2

(14.2) Xho I
(14.1) Bcl I
Bgl II (0.0/14.4)
(13.2) Bcl I
*(12.4) Cla I
(12.4) Eco RI
Pst I (2.1)
Pst I (2.4)
Sst I (2.6)
(11.6) Pst I
bla
pUC1078
Bcl I (3.8)
Sph I (3.9)
Sst I (5.2)
cat
(8.6) Sph I
(8.4) Bam HI
Xho I (7.0)
(8.0) Hind III
Eco RI (7.8)

*This restriction site is methylated in most *E. coli* hosts.

## FIG. 3

pUC1080

(16.1) Xho I
(16.0) Bcl I
(15.1) Bcl I
*(14.3) Cla I
(14.3) Eco RI
(13.5) Pst I

bla

Bgl II (0.0/16.3)

Pst I (2.1)
Pst I (2.4)
Sst I (2.6)

Bcl I (3.8)

Sph I (3.9)

Sst I (5.2)

(10.6) Sph I
(10.3) Bam HI
(9.7) Pst I
(9.5) Sst I
(9.0) Sst I
(8.4) Bam HI

vph

cat

Xho I (7.0)
Eco RI (7.8)
Hind III (8.0)

2-Sph I

*This restriction site is methylated in most *E. coli* hosts.

# FIG. 4

0117694

Digest both plasmids with *Cla* I and ligate the linear forms with T4 ligase. Transform *E. coli* host RRI and select ampicillin resistant tetracycline sensitive clones.

*This restriction site is methylated in most *E. coli* hosts.

# FIG. 5

Plasmid pUC1072 is linearized with *Cla* I. A *Taq* I fragment from pBR325 is isolated by preparative gel electrophoresis and ligated into the pUC1072 *Cla* I site. Chloramphenicol and ampicillin resistant transformants are selected in *E. coli* host RRI.

*This restriction site is methylated in most *E. coli* hosts.

# FIG. 6

Xho I    Bgl II

Cla I*

Eco RI

Pst I   bla

pUC1078

Pst I
Pst I

cat

Bam HI
Hind III
Eco RI    Xho I

vph   Pst I

Bam HI    Bam HI

Isolate viomycin resistance
gene by gel electrophoresis

*This *Cla* I site is
modified in *E.
coli.*

1) Linearize pUC1078 by
*Bam* HI digestion

2) Ligate linear pUC1078
with *vph Bam* HI
fragment

3) Transform *E. coli* RRI
selecting for viomycin,
chloramphenicol, and
ampicillin resistant clones

Xho I    Bgl II

*Cla I

Eco RI

Pst I   bla

pUC1080

Pst I
Pst I

vph   cat

Bam HI
Pst I      Xho I
Bam HI    Eco RI
Hind III

*This restriction site is methylated in most *E. coli* hosts.

# FIG. 7

(12.9) Sph I
(12.7) Bam HI
(12.4) Hind III
(12.4) Cla I
Sal I/Xho I (13.0)
Bgl II (0.0/13.2)

(11.8) Bcl I

(11.1)Eco RI

cat

(10.0) Pst I

bla

pUC1075

Pst I (2.1)
Pst I (2.4)
Sst I (2.6)

Bcl I (3.8)
Sph I (3.9)

Sst I (5.2)

Sal I/Xho I (7.0)

FIG. 8

pUC1079

0117694

# FIG. 9

1) Plasmid pBR325 is digested with endonuclease Sal I and plasmid pUC1061 is digested with endonuclease Xho I

2) The linear plasmid DNAs are mixed and ligated

3) E. coli host RRI is transformed selecting for ampicillin and chloramphenicol resistant transformants that are tetracycline sensitive

0117694

# FIG. 10

pUC1075

Labels (top circle): Bam HI, Sal I/Xho I, Hind III, Bgl II, Cla I, Bcl I, Eco RI, cat, bla, Bcl I, Sal I/Xho I

vph / Pst I / Bam HI / Bam HI

Isolate the vph gene on a
1.9 kb Bam HI fragment
by gel electrophoresis

1) Linearize pUC1075
   by Bam HI digestion

2) Ligate linear pUC1075
   with vph Bam HI fragment

3) Transform E. coli RRI selecting
   for viomycin, chloramphenicol,
   and ampicillin resistant clones

pUC1079

Labels (bottom circle): Sal I/Xho I, Bam HI, Bgl II, Bam HI, Hind III, Cla I, Bcl I, vph, cat, Eco RI, bla, Bcl I, Sal I/Xho I

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:
NRRL B-12014
NRRL B-12186
NRRL 15276
NRRL 15277
NRRL B-15278
NRRL B-15279
NRRL B-15280
NRRL B-15295
NRRL 11439
23724a
NRRL 12488
23724a
NRRL 15528
NRRL B-15527
NRRL B-15526

EPO Form 1165 07.81 e

**European Patent Office**

**EUROPEAN SEARCH REPORT**

Application number

## DOCUMENTS CONSIDERED TO BE RELEVANT

EP 84301049.7

| Category | Citation of document with indication, where appropriate, of relevant passages | Relevant to claim | CLASSIFICATION OF THE APPLICATION (Int. Cl. ³) |
|---|---|---|---|
| A | US - A - 4 338 400 (MANIS et al.)<br>* Abstract; claim 1 * | 1-10 | C 12 N 15/00//<br>C 12 R 1/19 |
| A | EP - A2 - 0 058 002 (THE UPJOHN COMPANY)<br>* Claims 7-10 * | 1-10 | |
| A | US - A - 4 362 817 (REUSSER)<br>* Abstract; claim 1 * | 1-10 | |
| A,D | US - A - 4 273 875 (MANIS)<br>* Abstract * | 4-6,9, 10 | |
| A | EP - A2 - 0 068 647 (THE UPJOHN COMPANY)<br>* Abstract * | 4-6,9, 10 | TECHNICAL FIELDS SEARCHED (Int. Cl. ³)<br><br>C 12 N<br>C 12 R |

The present search report has been drawn up for all claims

| Place of search | Date of completion of the search | Examiner |
|---|---|---|
| VIENNA | 29-05-1984 | WOLF |